# EUROPÄISCHE PATENTSCHRIFT

(11) **EP 1 311 322 B1**
(45) Veröffentlichungstag und Bekanntmachung des Hinweises auf die Patenterteilung: **09.03.2005**
(21) Anmeldenummer: 01964919.3
(22) Anmeldetag: 18.08.2001
(51) Int. Cl.: A61N 5/10

(54) **VORRICHTUNG ZUR BESTRAHLUNG VON GEWEBE**
DEVICE FOR IRRADIATING TISSUE
DISPOSITIF POUR L'IRRADIATION DE TISSU

(30) Priorität: 24.08.2000 DE 10041473
(43) Veröffentlichungstag der Anmeldung: 21.05.2003
(73) Patentinhaber: Müller, Reinhold G., 91080 Marloffstein (DE); Achterberg, Nils, 91233 Neunkirchen am Sand (DE)
(72) Erfinder: Müller, Reinhold G., 91080 Marloffstein (DE); Achterberg, Nils, 91233 Neunkirchen am Sand (DE)
(74) Vertreter: Hafner, Dieter, Dr. Dipl.-Phys.
(86) Internationale Anmeldenummer: PCT/DE2001/003167
(87) Internationale Veröffentlichungsnummer: WO 2002/015975

(56) Entgegenhaltungen:
- EP-A- 0 382 560
- DE-A- 19 604 789
- DE-A- 19 650 013
- US-A- 2 931 941
- PRICHARD B A: "A Proposed proton therapy facility at the ssc" NUCLEAR INSTRUMENTS AND METHODS IN PHYSICS RESEARCH, Bd. b79, Juni 1993 (1993-06), Seiten 895-897, XP001032074
- JÄKEL O; DEBUS J: "Selection of beam angles for radiotherapy of skull base tumours using charged particles" PHYSICS IN MEDICINE AND BIOLOGY, Bd. 45, Mai 2000 (2000-05), Seiten 1229-1241, XP002188971
- JONES W P ET AL: "DESIGN OF A BEAM TRANSPORT SYSTEM FOR A PROTON RADIATION THERAPY FACILITY" PROCEEDINGS OF THE 1999 PARTICLE ACCELERATOR CONFERENCE. NEW YORK CITY, NY, MARCH 27 - APRIL 2, 1999, PARTICLE ACCELERATOR CONFERENCE, NEW YORK, NY: IEEE, US, Bd. 4 OF 5 CONF. 18, 27. März 1999 (1999-03-27), Seiten 2519-2521, XP000895556 ISBN: 0-7803-5574-1

## Beschreibung

Die Erfindung betrifft eine Vorrichtung zur Bestrahlung von Gewebe mit den weiteren Merkmalen des Oberbegriffes des Patentanspruchs 1.

Mit der Realisierung der intensitätsmodulierten Strahlentherapie werden neue Behandlungsverfahren möglich. Ein vielversprechendes Konzept ist die sog. Tomotherapie, die den umfassendsten Versuch zur Optimierung der perkutanen (durch die Haut hindurch wirkenden) Tumortherapie mit ionisierenden Strahlen darstellt. Eine tumorkonforme Bestrahlung erzielt man durch spezielle Vorrichtungen, insbesondere Kollimatoren, zur Intensitätsmodulation und zur Eingrenzung des Photonenstrahls auf das Zielvolumen. Eine solche Vorrichtung ist z.B. im Dokument EP-A1-0 382560 offenbart.

Der Patient wird zur Bestrahlung auf einem Patiententisch in ein Therapiegerät eingefahren, in welchem durch Rotationen der Gantry eine Vielzahl von Einstrahlrichtungen eingestellt werden können. Die von einer Strahlenquelle erzeugte Strahlung wird auf einen oder mehrere Strahlerköpfe geführt. Wie beim klassischen Schnittbild-CT rotiert der Strahlerkopf um den Patienten und bestrahlt das zu behandelnde Gewebe Schicht für Schicht. Durch die gezielte Steuerung des Kollimators läßt sich die gewünschte Dosisverteilung im Patienten erzielen. Analog zum Spiral-CT kann der Patient auch relativ zum Strahlerkopf bewegt werden. Dabei beschreibt der Strahlerkopf eine Spiralbahn um den Patienten. Der Nachteil der bekannten Vorrichtungen besteht in dem hohen technologischen Aufwand bei der präzisen Rotation schwerer Massen sowie der problematischen Energie- und Informationsübertragung bei rotierenden Systemen. So entstehen z. B. Wartezeiten bis von einer Strahlrichtung auf die nächste umgestellt wird.

Der Erfindung liegt daher die Aufgabe zugrunde, eine Vorrichtung zur Bestrahlung von Gewebe mit den Merkmalen des Oberbegriffes des Patentanspruchs 1 derart auszubilden, daß die technische Zuverlässigkeit bei gleichzeitig einfachem Aufbau gesteigert wird und die bei der Strahlentherapie anfallenden Kosten reduziert werden.

Diese Aufgabe wird durch die kennzeichnenden Merkmale des Patentanspruchs 1 gelöst. Vorteilhafte Weiterbildungen der Vorrichtung ergeben sich aus den Unteransprüchen 2 bis 13.

Die Erfindung zeichnet sich zunächst dadurch aus, daß in dem Gehäuse der Vorrichtung eine Vielzahl von Strahlerköpfen feststehend angeordnet ist. Durch die ruhende Gantry bzw. die feststehenden Beschleunigerköpfe vermeidet man den hohen technologischen Aufwand, der zur präzisen Rotation des Systems notwendig ist. Außerdem wird das Problem von Energie- und Informationsübertragung bei rotierenden Systemen vermieden. Dadurch reduzieren sich die Geräteanschaffungs- und Betriebskosten beträchtlich und die technische Zuverlässigkeit steigt deutlich. Indem die Strahlerköpfe nicht mehr von einer Strahlrichtung auf die nächste umgestellt werden müssen, treten auch keine Wartezeiten mehr auf.

Außerdem ist gemäß der Erfindung für alle Strahlerköpfe eine gemeinsame Strahlenquelle vorgesehen, deren Strahlung auf die Strahlerköpfe aufteilbar ist. Ausgangspunkt ist demnach nur ein Primärstrahl. Diese Anordnung unterstützt den einfachen und kompakten Aufbau der erfindungsgemäßen Vorrichtung. Dazu kann mindestens ein Strahlungsteiler vorgesehen sein, welcher die Strahlung der Strahlungsquelle auf die einzelnen Strahlerköpfe aufteilt. Je nach Anforderung kann diese Aufteilung entweder simultan oder sequentiell erfolgen. Jeder Strahlerkopf kann also separat oder alle Strahlerköpfe gleichzeitig angesteuert werden. Dabei ist die Strahlenquelle als Elektronenstrahlquelle ausgebildet, wobei die Elektronen in der Kette Strahlenquelle, Strahlungsteiler, Umlenkelemente und Strahlerkopf beschleunigt, geführt und als Nutzstrahlung verwendbar sind.

Zur Strahlführung sind in der Gantry mindestens zwei Umlenkelemente vorgesehen, welche die Strahlung der Strahlenquelle umlenken und zu einem Strahlerkopf leiten. Die Umlenkelemente können auch derart angeordnet sein, daß sie die Strahlung mindestens eines Strahlerkopfes zur Bestrahlung des Zielvolumens umlenken. Das jeweilige Umlenkelement kann z. B. eine Umlenkung der Strahlung um 90° bewirken. Eine mit drei Umlenkelementen durchführbare 270°- Umlenkung des Strahls ist insofern vorteilhaft, als die Strahlführung doppelt fokussierend wirkt und zwar hinsichtlich der Strahlrichtung und der Strahlenergie. Durch die dreimalige Umlenkung wird zum einen eine lokale Fokussierungsverbesserung erreicht und zum anderen eine Energieschärfung. Die Teilchen, die dann aus dem dritten Umlenkelement heraustreten, haben im wesentlichen alle die gleiche Energie.

Erfindungsgemäß liegen die auf das Gewebe auftreffenden Strahlen auf mindestens zwei zueinander versetzten Ebenen. Dadurch ergibt sich eine Vielzahl von Möglichkeiten hinsichtlich der Patientenlagerung sowie der Bestrahlungsrichtungen.

Wegen der Maße der einzelnen Strahlerköpfe kann es zu Beschränkungen der Anordnungsmöglichkeiten in einer Ebene kommen, so daß die Strahlerköpfe auf mindestens zwei zueinander versetzten Ebenen angeordnet sind.

In Weiterbildung der Erfindung können die Elektronen auch auf ein Bremstarget geleitet werden, so daß die Elektronenstrahlung in harte Röntgenstrahlung umgewandelt wird. Dabei kann sowohl ein zylindrisches als auch ein sphärisches Bremstarget eingesetzt werden.

Zur Begrenzung des Querschnitts eines Strahls energiereicher Teilchen kann mindestens ein Strahlerkopf mit einem Multilamellenkollimator (MLK) versehen sein. Zur Realisierung einer intensitätsmodulierten Strahlenbehandlung können diese Mulitiamellenkollimatoren ansteuerbar sein.

Zweckmäßigerweise kann die Apertur des Nutzstrahls in wenigstens einer Richtung auf eine kleine Dimension (z. B. mm bis wenige cm) in Zentrumsebene einschränkbar sein. Diese Einschränkung der Apertur des Nutzstrahls ist entweder in transversaler Richtung (quer zur Patientenlängsachse) und/oder in longitudinaler Richtung (parallel zur Patientenlängsachse) möglich.

Vorteilhafterweise ist ein Patientenlagerungstisch vorgesehen, dessen Bewegung einer Bestrahlungsplanung entsprechend ansteuerbar ist. Auf diesem Wege läßt sich wegen der festen Anordnung der Strahlerköpfe eine Relativbewegung herbeiführen, so daß sich die benötigten Dosisverteilungen erzeugen lassen.

Der Patientenlagerungstisch kann dabei mittels einer digitalen Ansteuerung dem Ergebnis der Bestrahlungsplanung entsprechend kontinuierlich oder schrittweise bewegt werden.
Gegenüber mindestens eines Strahlerkopfes in Strahlrichtung auf der Austrittseite des Patienten kann ein Strahlungsdetektor, insbesondere ein Flächenstrahlungsdetektor angeordnet sein, der sowohl die bestrahlte Kontur des Feldes als auch die momentane Dosisleistung sowie die integral applizierte Dosis zweidimensional meßtechnisch erfaßt.

Zusammenfassend ist festzustellen, daß wegen der einfacheren Realisierbarkeit und der größeren technischen Zuverlässigkeit der vorliegenden Erfindung sich eine kostengünstige Alternative zu bereits bestehenden und in der Entwicklung befindlichen Technologien ergibt.

Die vorliegende Erfindung ist anhand von vorteilhaften Ausführungsbeispielen in den Zeichnungsfiguren näher erläutert. Diese zeigen:
- Fig. 1: ein Strahlführungsprinzip gemäß der vorliegenden Erfindung sowie
- Fig.2: eine räumliche Darstellung des Strahlführungsprinzips.

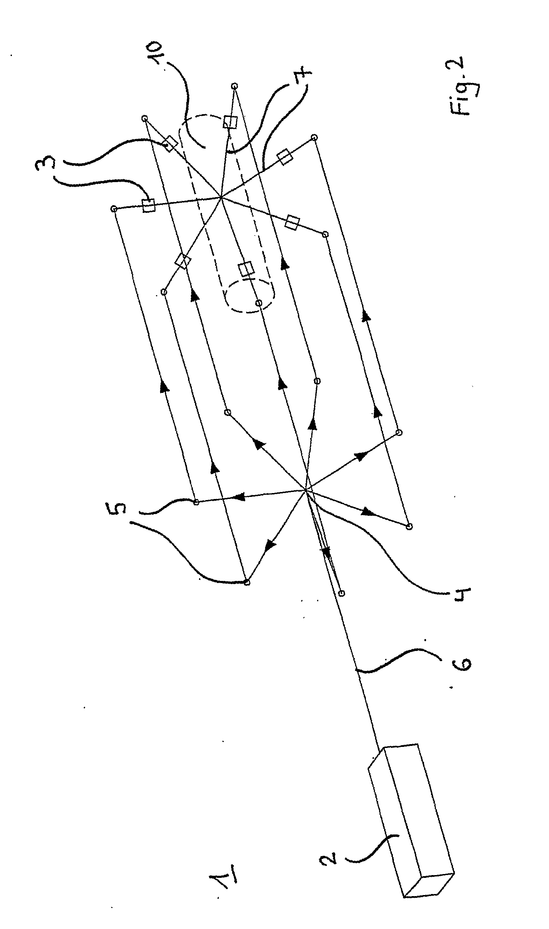

Die Bezugsziffer 1 bezeichnet die erfindungsgemäße Vorrichtung zur Bestrahlung von Gewebe in ihrer Gesamtheit. Die Vorrichtung dient insbesondere der Tumorbestrahlung eines auf dem Patientenlagerungstisch 8 liegenden Patienten 10. Die Vorrichtung 1 weist eine Strahlenquelle 2 auf, deren Primärstrahl 6 nach mehreren Umlenkungen auf einen Strahlerkopf 3 führt. Strahlenquelle 2 sowie Strahlerkopf 3 sind in einem Gehäuse (in den Zeichnungsfiguren nicht dargestellt) angeordnet, wobei die vom Strahlerkopf 3 abgegebene Strahlung auf das Gewebe des Patienten 10 trifft. Erfindungsgemäß ist eine Vielzahl von Strahlerköpfen 3 in dem Gehäuse feststehend angeordnet ( siehe Fig. 2). Dies hat gegenüber Vorrichtungen mit bewegten Strahlführungssystemen die Vorteile, daß sich die Geräteanschaffungs-und Betriebskosten erheblich reduzieren und die technische Zuverlässigkeit deutlich steigt.

Wie in Fig. 2 dargestellt, ist für alle Strahlerköpfe 3 eine gemeinsame Strahlenquelle 2 vorgesehen, deren Strahlung auf die Strahlerköpfe 3 aufgeteilt wird.

Mindestens ein Strahlungsteiler 4 dient der Aufteilung der Strahlung der Strahlungsquelle 2 auf die einzelnen Strahlerköpfe 3 bzw. die dazwischen angeordneten Umlenkelemente 5. Bei der Aufteilung der Strahlung kann jeder Strahlerkopf 3 separat angesteuert werden oder auch alle Strahlerköpfe 3 gleichzeitig. Die Umlenkelemente 5 dienen der Umlenkung der Strahlung der Strahlenquelle 2 und der Strahlungsführung zu dem jeweiligen Strahlerkopf 3.

Wie in den Zeichnungsfiguren dargestellt ist, bewirkt jedes Umlenkelement eine Umlenkung der Strahlung um 90°. Die gesamte Umlenkung des Strahls von 270° durch eine dreimalige Umlenkung ist insofern vorteilhaft, als sie doppelt fokussierend wirkt und zwar hinsichtlich der Strahlrichtung und der Strahlenergie.

Die Strahlerköpfe 3 sind auf versetzten Ebenen zueinander angeordnet, so daß aufgrund der Maße der Strahlerköpfe 3 keine räumlichen Anordnungsprobleme entstehen.

Die Strahlenquelle 2 stellt eine Elektronenstrahlenquelle dar. Die Elektronen werden in der Kette Strahlenquelle 2, Strahlungsteiler 4, Umlenkelemente 5 sowie Strahlerkopf 3 beschleunigt, geführt und schließlich als Nutzstrahlung verwendet. Mittels eines an den Strahlerkopf 3 angeordneten Bremstargets (zylindrisch oder sphärisch) kann die Elektronenstrahlung in harte Röntgenstrahlung umgewandelt werden. Zur Aufweitung des Nutzstrahls kann der Primärstrahl 6, also die primär beschleunigten geladenen Teilchen, mittels einer Abtastvorrichtung 11 ausgelenkt werden.

Mindestens einer der Strahlerköpfe 3 ist mit einem Multilamellenkollimator 12 versehen, welcher zur Begrenzung des Querschnitts des Nutzstrahls dient. Zur intensitätsmodulierten Strahlenbehandlung sind die Multilamellenkollimatoren 12 ansteuerbar.

Die Apertur der Nutzstrahlung, also die geladenen Teilchen oder auch die Bremsstrahlung, ist in wenigstens einer Richtung auf eine kleine Dimension im Millimeter- bis Zentimeter-Bereich in Zentrumsebene einschränkbar. Diese Einschränkung kann entweder in transversaler Richtung - quer zur Patientenlängsachse - und/oder in longitudinaler - parallel zur Patientenlängsachse - erfolgen.

Die Bewegung des Patientenlagerungstisches 8 ist entsprechend einer Bestrahlungsplanung ansteuerbar, damit das zu behandelnde Gewebevolumen vollkommen erfaßt wird. Dazu ist der Patientenlagerungstisch 8 um wenigstens eine Drehachse und/oder in seiner Längsachse bewegbar. Die Bewegung kann dabei kontinuierlich oder schrittweise erfolgen.

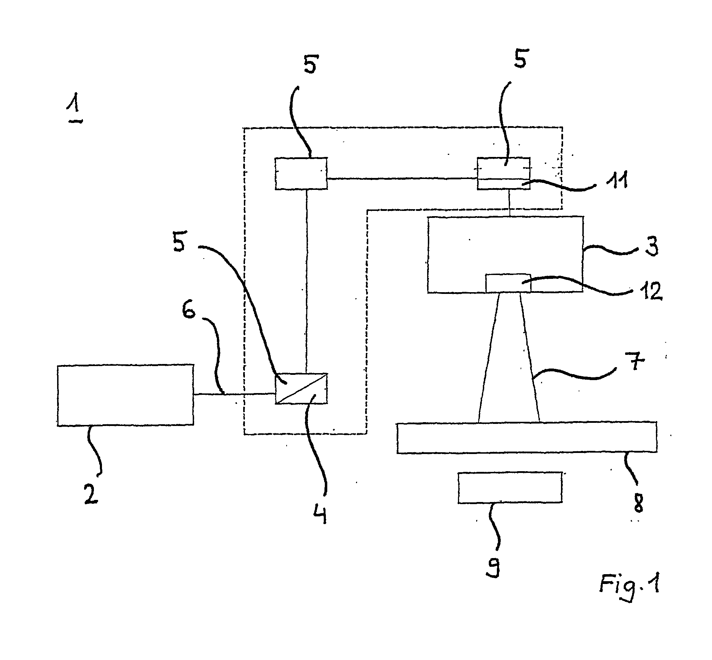

Gegenüber mindestens eines Strahlerkopfes 3 ist in Strahlrichtung auf der Austrittsseite des Patienten 10 ein Strahlungsdetektor 9, insbesondere ein Flächenstrahlungsdetektor, angeordnet, mit welchem sowohl die bestrahlte Kontur des Feldes als auch die momentane Dosisleistung sowie die integral applizierte Dosis zweidimensional meßtechnisch erfaßt werden kann (siehe Fig.1).

Ferner ist in der Vorrichtung 1 ein Mikroprozessor vorgesehen, der jedoch in den Zeichnungsfiguren nicht näher dargestellt ist. Über den Mikroprozessor erfolgt die Ansteuerung und Regelung von sämtlichen dynamischen Komponenten, wie Strahlungsquelle 2, Strahlungsteiler 4, Umlenkelemente 5 usw. Der Mikroprozessor dient außerdem dazu, die Ergebnisse eines Bestrahlungsplanungsprogramms umzusetzen.

## Patentansprüche

1. Vorrichtung (1) zur Bestrahlung von Gewebe, insbesondere von Tumoren, mit mindestens einer Strahlenquelle (2), deren Primärstrahl (6) auf mindestens einen Strahlerkopf (3) führt, wobei Strahlenquelle (2) sowie Strahlerkopf (3) in einem Gehäuse angeordnet sind und die vom Strahlerkopf (3) abgegebene Strahlung auf das Gewebe trifft,
**dadurch gekennzeichnet, daß**
a) eine Mehrzahl von Strahlerköpfen (3) in dem Gehäuse feststehend angeordnet ist,
b) für alle Strahlerköpfe (3) eine gemeinsame Strahlenquelle (2) vorgesehen ist, deren Strahlung durch einen Strahlungsteiler (4) auf die Strahlerköpfe (3) aufteilbar ist,
c) die Strahlungsquelle als Elektronenstrahlungsquelle ausgebildet ist,
d) zwischen dem Strahlungsteiler (4) und jedem Strahlerkopf (3) mindestens zwei Umlenkelemente (5) vorgesehen sind sowie
e) die auf das Gewebe auftreffenden Strahlen (7) auf mindestens zwei versetzten Ebenen liegen.

2. Vorrichtung nach Anspruch 1,
**dadurch gekennzeichnet, daß**
jeder Strahlerkopf (3) separat ansteuerbar ist.

3. Vorrichtung nach einem der vorhergehenden Ansprüche 1 oder 2,
**dadurch gekennzeichnet, daß**
alle Strahlerköpfe (3) gleichzeitig ansteuerbar sind.

4. Vorrichtung nach einem der vorhergehenden Ansprüche,
**dadurch gekennzeichnet, daß**
jedes Umlenkelement (5) eine Umlenkung der Strahlung um 90° bewirkt.

5. Vorrichtung nach einem der vorhergehenden Ansprüche,
**dadurch gekennzeichnet, daß**
die Strahlerköpfe (3) auf mindestens zwei versetzten Ebenen angeordnet sind.

6. Vorrichtung nach einem der vorhergehenden Ansprüche,
**dadurch gekennzeichnet, daß**
die Elektronen der Elektronenstrahlquelle beschleunigt, geführt und als Nutzstrahlung verwendbar sind.

7. Vorrichtung nach einem der vorhergehenden Ansprüchen,
**dadurch gekennzeichnet, daß**
die Elektronen mittels eines Bremstargets in den Strahlenköpfen (3) in harte Röntgenstrahlung umwandelbar sind.

8. Vorrichtung nach einem der vorhergehenden Ansprüche,
**dadurch gekennzeichnet, daß**
mindestens ein Strahlerkopf (3) mit einem Multilamellenkollimator (MLK) (12) versehen ist.

9. Vorrichtung nach Anspruch 8,
**dadurch gekennzeichnet, daß**
die Multilamellenkollimatoren (12) zur Realisierung einer intensitätsmodulierten Strahlenbehandlung ansteuerbar sind.

10. Vorrichtung nach einem der vorhergehenden Ansprüche,
**dadurch gekennzeichnet, daß**
die Apertur der Nutzstrahlung in wenigstens einer Richtung auf eine bestimmte Dimension in Zentrumsebene einschränkbar ist.

11. Vorrichtung nach einem der vorhergehenden Ansprüche,
**dadurch gekennzeichnet, daß**
ein Patientenlagerungstisch (8) vorgesehen ist, dessen Bewegung einer Bestrahlungsplanung entsprechend ansteuerbar ist.

12. Vorrichtung nach Anspruch 11,
**dadurch gekennzeichnet, daß**
der Patientenlagerungstisch (8) um wenigstens eine Drehachse und/ oder in seiner Längsachse bewegbar ist.

13. Vorrichtung nach einem der vorhergehenden Ansprüche,
**dadurch gekennzeichnet, daß**
gegenüber mindestens einem Strahlerkopf (3) in Strahlrichtung auf der Austrittsseite des Patienten ein Strahlungsdetektor (9), insbesondere ein Flächenstrahlungsdetektor angeordnet ist.

## Claims

1. Device (1) for irradiating tissue, in particular tumours, having at least one beam source (2), whose primary beam (6) leads to at least one radiating head (3), the beam source (2) and radiating head (3) being arranged in a housing and the radiation emitted by the radiating head (3) striking the tissue,
**characterised in that**
a) a plurality of radiating heads (3) are fixedly arranged in the housing,
b) a common beam source (2) is provided for all the radiating heads (3), the radiation of which beam source can be distributed to the radiating heads (3) by means of a radiation distributor (4),
c) the beam source is constructed as an electron radiation source,
d) at least two redirection elements (5) are provided between the radiation distributor (4) and each radiating head (3), and
e) the beams (7) which strike the tissue are located in at least two offset planes.

2. Device according to claim 1,
**characterised in that**
each radiating head (3) can be controlled separately.

3. Device according to either claim 1 or claim 2,
**characterised in that**
all the radiating heads (3) can be controlled simultaneously.

4. Device according to any one of the preceding claims,
**characterised in that**
each redirection element (5) brings about a redirection of the radiation through 90°.

5. Device according to any one of the preceding claims,
**characterised in that**
the radiating heads (3) are arranged in at least two offset planes.

6. Device according to any one of the preceding claims,
**characterised in that**
the electrons of the electron beam source are accelerated, guided and can be used as effective radiation.

7. Device according to any one of the preceding claims,
**characterised in that**
the electrons can be converted into hard X-rays by means of a retardation target in the radiating heads (3).

8. Device according to any one of the preceding claims
**characterised in that**
at least one radiating head (3) is provided with a multi-plate collimator (12).

9. Device according to claim 8,
**characterised in that**
the multi-plate collimators (12) can be controlled to produce intensity-modulated radiation therapy.

10. Device according to any one of the preceding claims,
**characterised in that**
the aperture of the effective radiation can be limited in at least one direction to a specific dimension in the centre plane.

11. Device according to any one of the preceding claims,
**characterised in that**
a patient-carrying table (8) is provided whose movement can be controlled in accordance with a radiation programme.

12. Device according to claim 11,
**characterised in that**
the patient-carrying table (8) can be moved about at least one axis of rotation and/or in the longitudinal axis thereof.

13. Device according to any one of the preceding claims,
**characterised in that**
a radiation detector (9), in particular a two-dimensional radiation detector, is arranged facing at least one radiating head (3) in the direction of radiation at the emergent side of the patient.

## Revendications

1. Dispositif (1) pour l'irradiation de tissu, en particulier de tumeurs, comportant au moins une source de rayonnement (2) dont le faisceau primaire (6) aboutit à au moins une tête d'irradiation (3), la source de rayonnement (2) ainsi que la tête d'irradiation (3) étant disposées dans un boîtier et le rayonnement émis par la tête d'irradiation (3) arrivant sur le tissu,
**caractérisé par le fait que**
a) plusieurs têtes d'irradiation (3) sont disposées à demeure dans le boîtier,
b) pour toutes les têtes d'irradiation (3), il est prévu une source de rayonnement (2) commune dont le rayonnement peut être réparti sur les têtes d'irradiation (3) par un séparateur de faisceau (4),
c) la source de rayonnement est constituée d'une source de rayonnement électronique,
d) il est prévu au moins deux éléments de déviation (5) entre le séparateur de faisceau (4) et chaque tête d'irradiation (3),
e) les rayons (7) arrivant sur le tissu sont situés dans au moins deux plans décalés.

2. Dispositif selon la revendication 1,
**caractérisé par le fait que**
chaque tête d'irradiation (3) peut être commandée séparément.

3. Dispositif selon l'une des revendications précédentes 1 ou 2,
**caractérisé par le fait que**
toutes les têtes d'irradiation (3) peuvent être commandées en même temps.

4. Dispositif selon l'une des revendications précédentes,
**caractérisé par le fait que**
chaque élément de déviation (5) provoque une déviation du rayonnement de 90°.

5. Dispositif selon l'une des revendications précédentes,
**caractérisé par le fait que**
les têtes d'irradiation (3) sont disposées dans au moins deux plans décalés.

6. Dispositif selon l'une des revendications précédentes,
**caractérisé par le fait que**
les électrons de la source de rayonnement électronique sont accélérés, guidés et utilisables comme faisceau utile.

7. Dispositif selon l'une des revendications précédentes,
**caractérisé par le fait que**
les électrons sont transformables en rayons X durs au moyen d'une cible de freinage dans les têtes d'irradiation (3).

8. Dispositif selon l'une des revendications précédentes,
**caractérisé par le fait que**
au moins une tête d'irradiation (3) est munie d'un collimateur multilames (MLK) (12).

9. Dispositif selon la revendication 8,
**caractérisé par le fait que**
le collimateur multilames (12) peut être commandé pour réaliser une radiothérapie d'intensité modulée.

10. Dispositif selon l'une des revendications précédentes,
**caractérisé par le fait que**
l'ouverture du faisceau utile dans au moins une direction peut être limitée à une dimension définie en plan central.

11. Dispositif selon l'une des revendications précédentes,
**caractérisé par le fait que**
il est prévu une table de traitement (8) dont le mouvement peut être commandé en fonction d'un planning d'irradiation.

12. Dispositif selon la revendication 11,
**caractérisé par le fait que**
la table de traitement (8) est déplaçable autour d'au moins un axe de rotation et/ou le long de son axe longitudinal.

13. Dispositif selon l'une des revendications précédentes,
**caractérisé par le fait que**
un détecteur de rayonnement (9), en particulier un détecteur de rayonnement surfacique est disposé en face d'au moins une tête d'irradiation (3) dans la direction du faisceau du côté de sortie du patient.
